# FASCICULE DE BREVET EUROPEEN

(11) **EP 4 061 449 B1**
(45) Date de publication et mention de la délivrance du brevet: **20.12.2023**
(21) Numéro de dépôt: 20816282.6
(22) Date de dépôt: 06.11.2020
(51) Int. Cl.: A61M 1/36, A61M 25/00

(54) **CANULE ARTÉRIELLE BIDIRECTIONNELLE POUR OXYGÉNATION PAR MEMBRANE EXTRACORPORELLE ET PROCÉDÉ D'UTILISATION D'UNE TELLE CANULE**
BIDIREKTIONALE ARTERIELLE KANÜLE ZUR EXTRAKORPORALEN MEMBRANOXYGENIERUNG UND VERFAHREN ZUR VERWENDUNG EINER SOLCHEN KANÜLE
BIDIRECTIONAL ARTERIAL CANNULA FOR EXTRACORPOREAL MEMBRANE OXYGENATION AND METHOD FOR USING SUCH A CANNULA

(30) Priorité: 18.11.2019 FR 1912831
(43) Date de publication de la demande: 28.09.2022
(73) Titulaire: Novaflow, 92420 Vaucresson (FR)
(72) Inventeur: COUETIL, Jean-Paul, 92420 VAUCRESSON (FR)
(74) Mandataire: Germain Maureau
(86) Numéro de dépôt international: PCT/FR2020/052022
(87) Numéro de publication internationale: WO 2021/099716

(56) Documents cités:
- WO-A1-2016/137212
- FR-A1- 3 058 642
- US-A1- 2012 259 273
- US-A1- 2016 121 079

## Description

### Domaine Technique

La présente invention concerne une canule artérielle pour oxygénation par membrane extracorporelle (ECMO) et un procédé d'utilisation d'une telle canule.

### Technique antérieure

L'oxygénation par membrane extracorporelle (ECMO) désigne une méthode d'assistance circulatoire d'urgence pour un patient victime d'un choc cardiogénique. Cette méthode, en cas de défaillance circulatoire aigüe, est utilisée au lit du patient en réanimation médico-chirurgicale et quel que soit la raison de la défaillance cardiaque. C'est une méthode de circulation extracorporelle, utilisée également pour réaliser des interventions de chirurgie cardiaque à ciel ouvert. Dans la suite de la description, on parlera plus précisément d'oxygénation par membrane extracorporelle Veino-Artérielle (VA), permettant d'assister à la fois le coeur et les poumons.

L'objectif de l'ECMO VA est d'aspirer le sang pauvre en oxygène hors du corps du patient, de le charger en oxygène, puis de le réinjecter dans le corps du patient. Le sang est aspiré au niveau de l'oreillette droite (OD) du coeur du patient ou de sa veine cave inférieure par l'intermédiaire d'une canule veineuse, nommée également canule d'admission. Il traverse ensuite un oxygénateur. Puis il est réinjecté de manière rétrograde, c'est-à-dire vers le coeur, dans l'artère iliaque ou dans l'artère fémorale du patient via une canule artérielle, nommée également canule de réinjection. Une pompe centrifuge tournant à une vitesse constante, de l'ordre de 3000 tr/min, permet cette circulation extracorporelle de sang. La vitesse de rotation de la pompe permet d'obtenir des débits variables adaptés aux besoins du patient.

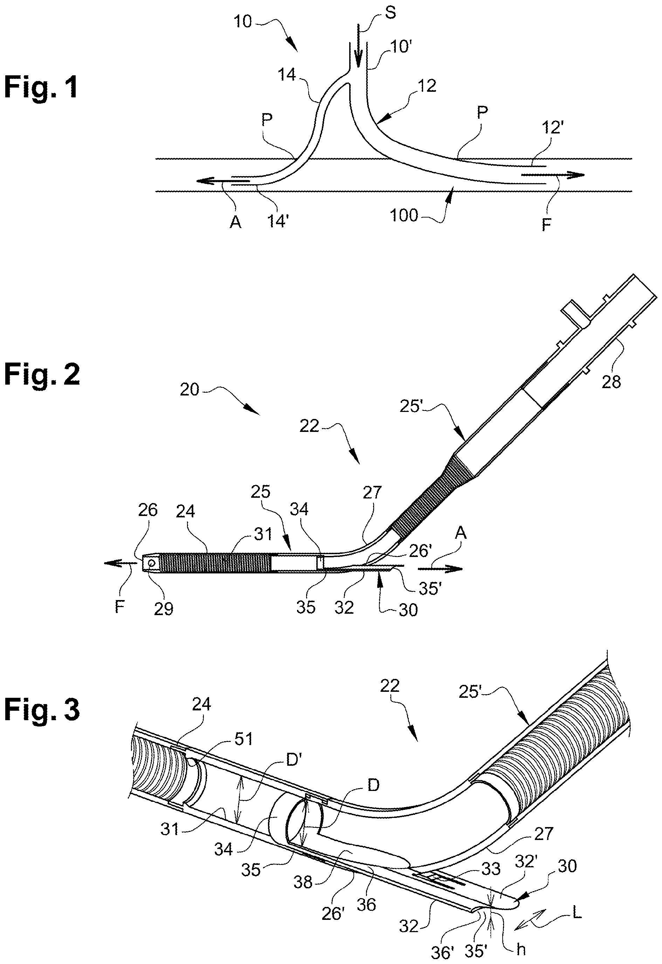

L'inconvénient majeur de cette procédure est que l'on observe une ischémie du membre inférieur due à la non perfusion du membre inférieur. En effet, la canule artérielle obstrue l'artère dans laquelle le sang est réinjecté après oxygénation et empêche donc le sang de circuler en direction du membre inférieur. Il est alors nécessaire de dériver une partie du sang oxygéné par l'oxygénateur, pour l'envoyer dans l'artère fémorale vers le membre inférieur, c'est-à-dire dans le sens antérograde, en aval du point de pénétration de la canule artérielle par rapport au sens antérograde de circulation du sang. Cette dérivation est généralement réalisée par une canule ou un cathéter de rétro-perfusion telle qu'illustrée à la figure 1. Cela implique une manipulation délicate et souvent réalisée de manière aléatoire souvent avec difficulté, pour introduire la canule de rétro-perfusion dans l'artère fémorale.

La figure 1 représente une canule artérielle 10 selon l'art antérieur, comportant une canule artérielle principale 12 et une canule de rétro-perfusion 14. Les canules artérielle principale 12 et de rétro-perfusion 14 comprennent une première extrémité, respectivement 12' et 14', introduite dans une artère 100 d'un patient, comme par exemple l'artère fémorale, et sont reliées entre elles par leur extrémité opposée 10', pour former la canule artérielle 10. Les canules artérielle principale 12 et de rétro-perfusion 14 sont introduites dans l'artère 100 grâce à des mandrins d'introduction (non représentés) qui permettent de dilater l'artère. Les mandrins sont ensuite retirés pour permettre au sang de circuler dans les canules artérielle principale 12 et de rétro-perfusion 14. La canule de rétro-perfusion 14 est introduite dans l'artère 100 au niveau d'un point de pénétration aval « p », situé en aval d'un point de pénétration amont P de la canule artérielle principale 12, par rapport au sens de circulation antérograde du sang, du coeur vers les membres inférieurs, représenté par la flèche A. La canule artérielle 10 est reliée à un oxygénateur (non représenté) pour permettre de réaliser une oxygénation par membrane extracorporelle (ECMO). L'oxygénateur est lui-même en communication avec une canule veineuse (non représentée) qui permet d'aspirer du sang S au niveau de l'oreillette droite ou de la veine cave inférieure (non représentée) du coeur du patient. Lors de l'ECMO, le sang S circule depuis la canule veineuse (non représentée), dans l'oxygénateur (non représenté), puis en parallèle dans la canule artérielle principale 12 et dans la canule de rétro-perfusion 14. A partir de la canule artérielle principale 12, le sang est réinjecté de manière rétrograde selon la flèche F dans l'artère 100, c'est-à-dire selon le sens de circulation rétrograde, en direction de l'artère iliaque. A partir de la canule de rétro-perfusion 14 le sang est réinjecté dans l'artère 100, dans le sens de circulation antérograde A, en direction des membres inférieurs. La canule artérielle principale 12 étant occlusive pour l'artère 100, la canule de rétro-perfusion 14 permet d'alimenter en sang le membre inférieur.

Une autre canule bidirectionnelle est présentée dans le document US2016/121079.

La présente invention a notamment pour but de résoudre les inconvénients précités.

### Exposé de l'invention

L'invention a pour objet une canule artérielle pour ECMO comprenant :
- une canule artérielle principale comportant une paroi interne et présentant une première extrémité avec un orifice de sortie de sang, la première extrémité étant destinée à être introduite dans une artère afin d'injecter le sang de manière rétrograde dans l'artère, et
- une canule de rétro-perfusion configurée pour être mobile en translation entre une position rétractée dans la première extrémité de la canule artérielle principale et une position déployée au moins en partie hors de première extrémité de la canule artérielle principale, à l'opposé de l'orifice de sortie de sang de la canule artérielle principale.

Ainsi, la canule artérielle selon l'invention permet d'injecter du sang dans l'artère de manière rétrograde, vers le coeur, et de manière antérograde, vers le membre inférieur, afin d'éviter le risque d'ischémie et de thrombose engendrées par l'ECMO, tout en évitant une opération délicate d'introduction d'une canule de rétro-perfusion dans une artère fémorale.

Selon d'autres caractéristiques de l'invention, la canule de l'invention comporte l'une ou plusieurs des caractéristiques optionnelles suivantes considérées seules ou selon toutes les combinaisons possibles.

Selon une caractéristique, la canule artérielle principale présente une forme en L avec une première branche correspondant à la première extrémité destinée à être introduite dans l'artère, et une seconde branche destinée à coopérer avec un oxygénateur, la première branche et la seconde branche étant reliées par un coude.

Selon une caractéristique, le coude présente un angle de courbure compris entre 90° et 150°, et de préférence de 135°. De cette façon, la canule de rétro-perfusion est aisément déployée hors de la canule artérielle principale.

Selon une caractéristique, non optionnelle et faisant partie de l'invention, la canule de rétro-perfusion comprend un tube de diamètre inférieur au diamètre de la canule artérielle principale et une bague de diamètre sensiblement identique au diamètre de la canule artérielle principale, disposée à une extrémité proximale de la canule de rétro-perfusion par rapport à l'orifice de sortie du sang de la canule artérielle principale. Ainsi, la canule de rétro-perfusion est guidée lors de son déploiement hors de la canule artérielle principale, et lorsque la canule de rétro-perfusion est en position déployée, elle est retenue à la canule artérielle principale par sa bague coopérant avec la paroi interne de la canule artérielle principale, afin d'empêcher tout désaxement ou déboitement pouvant entraîner une hémolyse ou un traumatisme artériel.

Selon une caractéristique, la canule de rétro-perfusion est configurée pour glisser en translation au contact de la paroi interne de la canule artérielle principale, jusqu'à ce que la bague entre en contact avec une butée de la paroi interne.

Selon une caractéristique, une découpe est présente entre le tube et la bague. Cette découpe est configurée pour coopérer avec le coude de la canule artérielle principale. En outre, cette découpe peut être configurée pour coopérer avec un mandrin d'introduction configuré pour induire le déploiement de la canule de rétro-perfusion et pour optimaliser le flux de circulation du sang dans canule artérielle principale lorsque la canule de rétro-perfusion est en position déployée.

Selon une caractéristique, le tube présente une extrémité distale biseautée, à l'opposé de la bague, de sorte à être adaptée à la courbure du coude de la canule artérielle principale lorsque la canule de rétro-perfusion est en position rétractée. Ainsi, en position rétractée, l'extrémité distale du tube est complémentaire du coude et ne dépasse pas du coude, ni de l'intérieur ni de l'extérieur de celui-ci, ce qui permet un glissement aisé de la canule artérielle hors de l'artère lors de son retrait.

Selon une caractéristique, le tube comporte une ouverture dirigée vers la seconde branche de la canule artérielle principale. L'ouverture permet de s'assurer que le sang peut circuler dans le tube, même si l'extrémité distale biseautée est obstruée. En effet, l'extrémité distale biseautée peut être obstruée par exemple par l'artère.

Selon une caractéristique, l'extrémité distale biseautée présente une paroi comportant une première face de longueur supérieure à une deuxième face opposée, la première face comprenant l'ouverture dirigée vers la seconde branche de la canule artérielle principale.

Selon une caractéristique, le tube présente une extrémité proximale biseautée, à proximité de la découpe, de sorte à être adaptée à la courbure du coude de la canule artérielle principale lorsque la canule de rétro-perfusion est en position déployée. Ainsi, en position déployée, l'extrémité proximale du tube est complémentaire du coude et ne dépasse pas de l'intérieur du coude, ce qui permet une circulation aisée du sang dans la canule artérielle principale.

Selon une caractéristique, le tube présente un profil elliptique, c'est-à-dire une section transversale elliptique. Ainsi, la hauteur et l'encombrement de la canule de rétro-perfusion en position rétractée à l'intérieur de la première extrémité de la canule artérielle principale ou en position déployée dans l'artère sont minimisés.

Selon une caractéristique, le tube de la canule de rétro-perfusion présente une surface de l'ordre de 3 à 3,5 mm2 et de préférence de 3,15 mm2, avec une hauteur de l'ordre de 1 à 1,5 mm et de préférence de 1,2 mm et une largeur de l'ordre de 3 à 3,5 mm et de préférence de 3,34 mm. Ainsi, un débit de sang minimum de 1 l/min est apte à circuler dans le tube. Un tel débit est suffisant pour prévenir l'ischémie du membre inférieur.

Selon une caractéristique, la bague présente une paroi intérieure convexe. Ainsi, le passage de la position déployée à la position rétractée de la canule de rétro-perfusion est facilité. En outre, cela permet de laminer le flux sanguin et ainsi d'éviter l'hémolyse et les flux turbulents.

Selon une caractéristique, la canule artérielle principale présente une rainure longitudinale au niveau de sa paroi interne coopérant avec une nervure disposée sur la bague de la canule de rétro-perfusion. De cette façon, la mobilité en translation de la canule de rétro-perfusion dans la canule artérielle principale est guidée. En outre, la rainure forme une butée pour la nervure lors de la translation de la canule de rétro-perfusion, et cela permet à la bague d'être disposée au contact de la paroi interne de la canule artérielle principale, de manière régulière, sans qu'une butée fasse saillie dans la canule artérielle principale, et ainsi d'éviter les flux turbulents et les risques d'hémolyse.

Selon une caractéristique, la rainure de la canule artérielle principale présente une longueur correspondant à la longueur de translation de la canule de rétro-perfusion. Ainsi, la rainure limite la translation de la canule de rétro-perfusion lors du passage entre les positions de retrait et déployée.

En variante, la canule artérielle principale présente une portion de translation de la bague, la portion de translation présentant un diamètre interne supérieur au diamètre interne du reste de la canule artérielle principale. Ainsi, la bague coopère en translation avec cette portion de translation et entre en butée avec le reste de la canule artérielle principale, présentant un diamètre inférieur Ainsi la translation de la canule de rétro-perfusion est limitée par un effet de butée de la bague procuré par la différence de diamètre interne de la canule principale, de sorte que l'extrémité distale du tube affleure le coude lorsque la canule de rétro-perfusion est en position rétractée, et l'extrémité proximale du tube affleure le coude en position déployée.

Selon une caractéristique, la canule artérielle principale présente une lumière dans sa paroi opposée à l'orifice de sortie du sang, de préférence de diamètre sensiblement identique au diamètre du tube de la canule de rétro-perfusion, pour permettre le déploiement du tube dans l'artère. Ainsi, la canule de rétro-perfusion est facilement déployée hors de la canule artérielle principale.

Selon une caractéristique, la lumière est disposée dans le coude de la canule artérielle principale.

Selon une caractéristique, la paroi interne de la canule artérielle principale au contact de la canule de rétro-perfusion en position rétractée, est en matériau biocompatible rigide, tel qu'en matériau métallique ou en polycarbonate. Ainsi, la canule de rétro-perfusion est apte à coulisser aisément dans la canule artérielle principale sans que celle-ci ne puisse être déformée lors de sa manipulation. En outre, la canule artérielle principale est peu encombrante de sorte qu'elle perturbe au minimum le flux sanguin.

Selon une caractéristique, la canule artérielle principale, en dehors de la paroi interne au contact de la bague et du tube de la canule de rétro-perfusion, est en matériau souple, tel que le nitinol ou l'acier tressé de préférence surmoulé d'un matériau plastique du type silicone ou polyuréthane. Ainsi, la canule artérielle principale est atraumatique et est aisément introduite dans l'artère.

En outre, le matériau souple est apte à être déformé pour permettre de conférer toute forme à la canule artérielle principale, notamment une forme en Z avec une première branche libre correspondant à la première extrémité destinée à être introduite dans l'artère, une branche centrale et une seconde branche libre destinée à coopérer avec un oxygénateur. Cette forme en Z permet de diminuer l'encombrement spatial du montage et de fixer plus facilement la canule à la peau d'un patient en évitant une plicature de la canule.

Selon une caractéristique, la canule de rétro-perfusion présente une paroi d'épaisseur de l'ordre de 1 mm maximum. Ainsi, l'encombrement est faible.

Selon une caractéristique, la bague de la canule de rétro-perfusion est en plastique rigide, tel que le polyuréthane, ou en métal rigide biocompatible tel que l'acier. Ainsi, le guidage de la translation de la canule de rétro-perfusion est optimisé.

Selon une caractéristique, le tube de la canule de rétro-perfusion est en matériau composite à base de métal et plastique à son extrémité proximale et en plastique souple tel que le silicone, à son extrémité distale. Ainsi, la canule de rétro-perfusion est atraumatique à son extrémité distale, et non déformable dans son extrémité proximale.

Selon une caractéristique, le tube et la bague sont reliés par un insert métallique formant la découpe, tel qu'un feuillard en acier. Ainsi, la canule de rétro-perfusion présente une bonne résistance mécanique.

Selon une caractéristique, la première extrémité de la canule artérielle principale comporte au moins un trou dans sa paroi, à proximité de son orifice de sortie du sang. Ainsi, le sang peut s'évacuer en cas d'obturation de l'orifice de sortie.

L'invention concerne en outre un mandrin d'introduction configuré pour être inséré dans la canule artérielle telle que décrit précédemment, afin de permettre à la fois l'insertion de la canule artérielle principale dans l'artère et le déploiement de la canule de rétro-perfusion hors de la canule artérielle principale, le mandrin d'introduction étant configuré pour obstruer la canule artérielle principale. Ainsi, aucun flux de sang ne circule tant que le mandrin d'introduction est disposé dans la canule artérielle principale.

Selon une caractéristique le mandrin d'introduction est configuré pour être obstructif dans la première branche de la canule artérielle principale et non obstructif dans le coude et la deuxième branche de cette canule artérielle principale. Ainsi, lorsque le coude est positionné dans l'artère, du sang est apte à s'introduire dans la canule artérielle principale via la lumière dans sa paroi opposée à l'orifice de sortie du sang, et le sang est apte à remonter dans la deuxième branche. Un opérateur peut alors identifier que le coude est dans l'artère et donc que la canule artérielle est dans une bonne position.

Selon une caractéristique, le mandrin d'introduction présente une forme complémentaire de la canule artérielle principale avec la canule de rétro-perfusion en position rétractée.

Le mandrin d'introduction est configuré pour être inséré dans la canule artérielle principale comportant la canule de rétro-perfusion en position rétractée, sans entrer en contact avec le tube de la canule de rétro-perfusion, et configuré pour coopérer avec la bague de la canule de rétro-perfusion pour l'entrainer en déploiement lors de son retrait. En outre, le mandrin d'introduction permet de dilater l'artère pour faciliter l'introduction de la canule artérielle principale dans l'artère.

Selon une caractéristique, le mandrin d'introduction comporte une extrémité proximale configurée pour être disposée dans la première extrémité de la canule artérielle principale, l'extrémité proximale présentant un diamètre croissant jusqu'à un diamètre sensiblement identique au diamètre de la canule artérielle principale, l'extrémité proximale étant suivie d'une première portion intermédiaire de longueur au moins supérieure à celle de la canule de rétro-perfusion et de diamètre inférieur au diamètre de la canule artérielle principale, la première portion intermédiaire étant suivie d'une deuxième portion intermédiaire de diamètre sensiblement identique à celui de la canule artérielle principale.

Selon une caractéristique, le mandrin d'introduction comporte un évidement complémentaire de la découpe de la canule de rétro-perfusion, et l'évidement comporte un ergot configuré pour entraîner la canule de rétro-perfusion en translation hors de la canule artérielle lorsque le mandrin d'introduction est retiré de la canule artérielle principale.

Selon une caractéristique, le mandrin d'introduction comporte une gorge dans laquelle la bague est destinée à se loger. Ainsi, la canule de rétro-perfusion est apte à être maintenue en position rétractée dans la canule artérielle principale lors de l'insertion de la canule artérielle principale dans une artère, et apte à être entrainée dans sa position déployée lors du retrait du mandrin d'introduction de la canule artérielle.

Selon une caractéristique, la gorge est complémentaire de la paroi interne convexe de la bague, de sorte à former un système semi-rétentif élastique lorsque la paroi interne convexe de la bague se loge dans la gorge du mandrin d'introduction. Ainsi, la canule de rétro-perfusion est apte à être maintenue en position rétractée dans la canule artérielle principale lors de l'insertion de la canule artérielle principale dans une artère, et apte à être entrainée dans sa position déployée lors du retrait du mandrin d'introduction de la canule artérielle. Lorsque la bague vient en butée lors du déploiement de la canule de rétro-perfusion, le mandrin d'introduction seul continue sa translation par un effet de traction exercé sur lui par un opérateur.

Selon une caractéristique, le mandrin d'introduction est perforé axialement en son centre en un cylindre creux permettant le passage d'un guide.

L'invention concerne également un mandrin de retrait configuré pour être inséré dans la canule artérielle telle que décrite précédemment, afin de permettre de rétracter la canule de rétro-perfusion dans la canule artérielle principale, et de permettre de retirer la canule artérielle principale hors de l'artère, le mandrin de retrait étant configuré pour obstruer la canule artérielle principale.

Selon une caractéristique, le mandrin de retrait est perforé axialement en son centre en un cylindre creux permettant le passage d'un guide.

Selon une caractéristique, le mandrin de retrait comprend un renflement configuré pour coopérer avec la bague de la canule de rétro-perfusion afin de l'entrainer en translation dans la canule artérielle principale, lorsque le mandrin de retrait est inséré dans la canule artérielle principale.

L'invention concerne également un kit comprenant une canule artérielle, un mandrin d'introduction et un mandrin de retrait, tels que décrit précédemment.

### Brève description des dessins

[Fig. 1] La figure 1 est une vue schématique d'une canule artérielle principale et d'une canule de rétro-perfusion selon l'art antérieur.
[Fig. 2] La figure 2 représente une canule artérielle pour ECMO selon le présent exposé, en coupe longitudinale.
[Fig. 3] La figure 3 est une vue schématique en coupe longitudinale d'une partie de la canule artérielle de la figure 2.
[Fig. 4] La figure 4 est une vue schématique en perspective d'une canule de rétro-perfusion de la canule artérielle de la figure 2.
[Fig. 5] La figure 5 est une vue détaillée de la figure 3, illustrant l'alignement entre la canule de rétro-perfusion et la canule artérielle principale lorsque la canule de rétro-perfusion est en position déployée.
[Fig. 6] La figure 6 est une vue schématique en coupe transversale de la canule artérielle comprenant une canule de rétro-perfusion.
[Fig. 7] La figure 7 est une vue schématique en perspective d'un mandrin d'introduction selon un premier mode de réalisation.
[Fig. 8] La figure 8 est une vue schématique en perspective d'un détail de mandrin d'introduction de la figure 7.
[Fig. 9] La figure 9 est une vue schématique en coupe du mandrin d'introduction de la figure 8, inséré dans la bague de la canule de rétro-perfusion selon le présent exposé.
[Fig. 10] La figure 10 est une vue schématique en coupe longitudinale d'une partie de canule artérielle munie d'un mandrin d'introduction selon la figure 7.
[Fig.11] La figure 11 est une autre vue schématique en coupe longitudinale de la canule artérielle, illustrant un mode de réalisation selon lequel la canule artérielle principale présente une différence de diamètre interne.de la figure 10
[Fig. 12] La figure 12 est une vue schématique partielle d'un mandrin d'introduction selon un deuxième mode de réalisation.
[Fig. 13] La figure 13 est une vue schématique du mandrin d'introduction de la figure 9 inséré dans une canule de rétro-perfusion d'une canule artérielle selon le présent exposé.
[Fig. 14] La figure 14 est une vue schématique partielle de la canule artérielle selon le présent exposé, munie d'un mandrin de retrait inséré dans la canule principale.
[Fig. 15] La figure 15 est une vue schématique en coupe longitudinale d'une partie de la canule artérielle de la figure 14, détaillant le mandrin de retrait.
[Fig. 16] La figure 16 est une vue schématique en perspective du mandrin de retrait des figures 14 et 15.
[Fig. 17] La figure 17 est une vue schématique en perspective d'une partie de la canule artérielle de la figure 2, comportant une canule de rétro-perfusion selon une variante de réalisation.
[Fig. 18] est une vue schématique en perspective d'une partie de la canule de rétro-perfusion illustrée à la figure 17.

### Description des modes de réalisation

La figure 1 est décrite précédemment.

La figure 2 illustre une canule artérielle 20 selon le présent exposé.

La canule artérielle 20 présente une canule artérielle principale 22 présentant une première extrémité 24 comprenant un orifice de sortie 26 de sang, et une deuxième extrémité 28 destinée à être reliée à un oxygénateur (non représenté).

La canule artérielle principale 22 peut présenter une forme de L, dont une première branche 25 comprend la première extrémité 24, et une seconde branche 25' comprend la deuxième extrémité 28. Les première 25 et seconde 25' branches peuvent être reliées par un coude 27 qui peut présenter un angle de courbure de 135° en moyenne et pouvant varier de 90° à 150°.

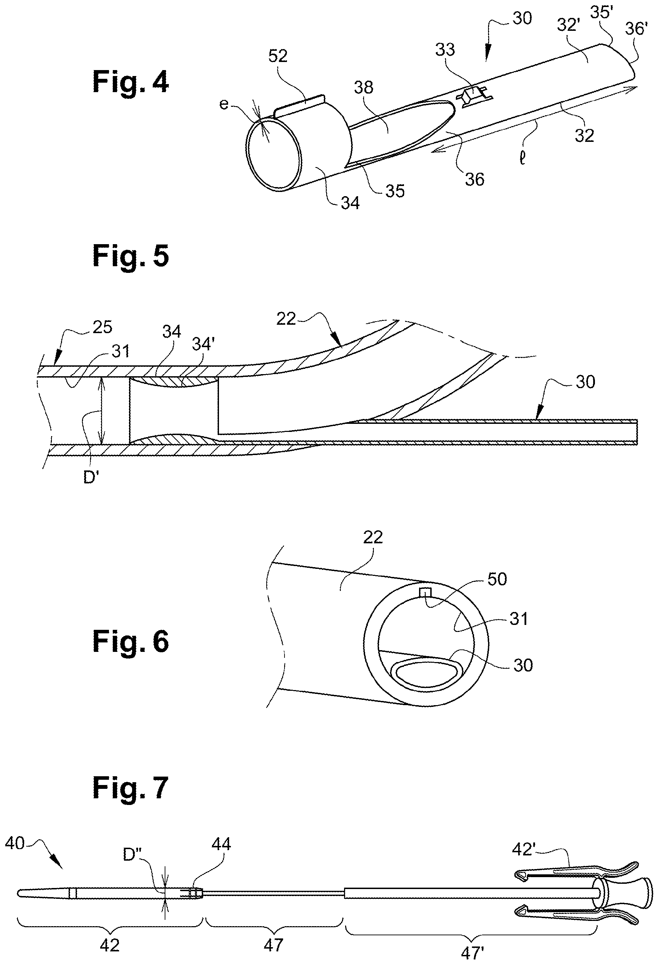

La première extrémité 24 est configurée pour être introduite dans une artère (non représentée) afin d'injecter le sang de manière rétrograde selon la flèche F, dans l'artère. Elle comporte une canule de rétro-perfusion 30 configurée pour être mobile en translation entre une position rétractée (figure 6) dans la première extrémité 24 de la canule artérielle principale 22 et une position déployée (figure 2) dans l'artère, partiellement hors de la canule artérielle principale 22, à l'opposé de l'orifice de sortie 26 de sang de la canule artérielle principale 22. La canule de rétro-perfusion 30 est destinée à injecter du sang dans l'artère de manière antérograde, selon la flèche A.

La première extrémité 24 de la canule artérielle principale 22 peut être rectiligne, de même que la canule de rétro-perfusion 30.

La canule artérielle principale 22 peut comporter au niveau de son extrémité proximale à l'orifice de sortie 26 de sang, un trou 29 permettant d'évacuer le sang en cas d'obturation de l'orifice de sortie 26.

La canule artérielle principale 22 peut être principalement formée en nitinol ou en acier tressé surmoulé de silicone ou de polyuréthane. Elle peut présenter une portion au contact de la canule de rétro-perfusion 30 en position rétractée, en polycarbonate. Ainsi, elle peut présenter une partie souple en nitinol ou en acier, et une partie rigide en polycarbonate.

La canule de rétro-perfusion comprend une extrémité proximale 35 par rapport à l'orifice de sortie 26 du sang de la canule artérielle principale, et une extrémité distale 35' opposée.

Comme l'illustre plus en détail les figures 3 à 6, la canule de rétro-perfusion 30 peut comprendre un tube 32 tel qu'un tube elliptique, présentant par exemple une surface de 3,15 mm2, une hauteur « h » de 1,2 mm, une largeur L de 3,34 mm et une longueur « l » de l'ordre de 5 à 15 mm. La canule de rétro-perfusion 30 peut comprendre également une bague 34 telle qu'une bague circulaire. La bague 34 peut être métallique ou en polyuréthane. Elle peut présenter un diamètre D très légèrement inférieur au diamètre interne D' de la canule artérielle principale permettant à la canule de rétro-perfusion de glisser sans difficulté dans la canule artérielle principale. Le tube 32 peut comprendre une extrémité proximale 36 par rapport à l'orifice de sortie 26 du sang de la canule artérielle principale 22, et une extrémité opposée dite distale 36'. La bague 34 peut être disposée à l'extrémité proximale 35 de la canule de rétro-perfusion30. Elle peut être séparée du tube 32 par une découpe 38. Les extrémités proximale 36 et distale 36' du tube 32 peuvent être obliques, c'est-à-dire biseautées.

L'extrémité distale 36' biseautée, peut être adaptée à la courbure du coude 27 de la canule artérielle principale lorsque la canule de rétro-perfusion 30 est en position rétractée. Ainsi, en position rétractée, l'extrémité distale 36' du tube 32 est complémentaire du coude 27. Elle ne dépasse pas du coude 27, ni de l'intérieur ni de l'extérieur de celui-ci, ce qui permet un glissement aisé de la canule artérielle hors de l'artère lors de son retrait. L'extrémité distale 36' biseautée forme un orifice de passage du sang configuré pour diriger le sang à l'opposé de la seconde branche 25' de la canule artérielle principale 22.

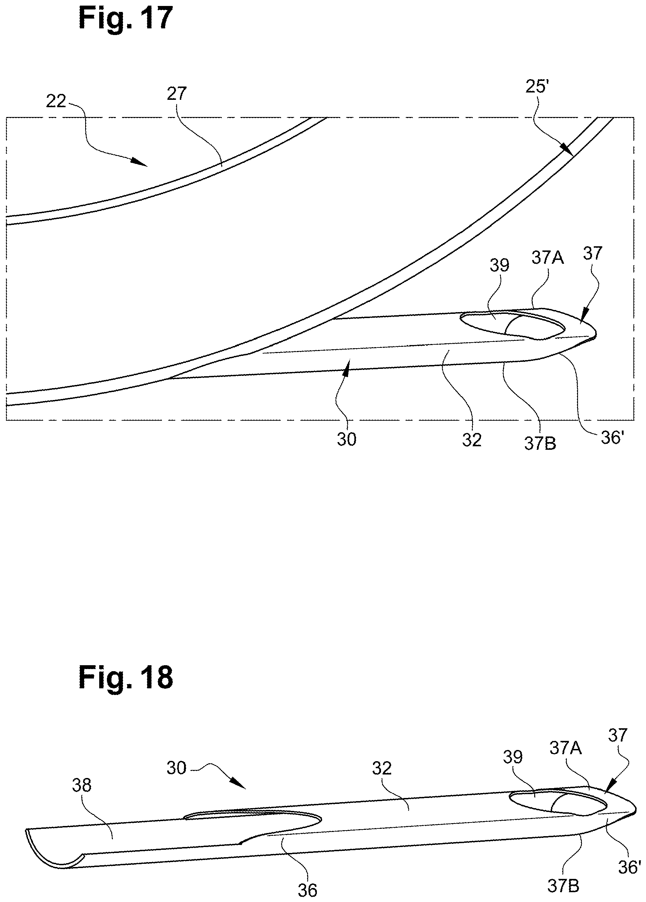

Comme le montrent les figures 17 et 18, le tube 32 peut comporter une ouverture 39 dirigée vers la seconde branche 25' de la canule artérielle principale 22. L'ouverture peut être disposée au niveau de l'extrémité distale 36' du tube 32.

L'extrémité distale 36' biseautée peut présenter une paroi 37 comportant une première face 37A de longueur supérieure à une deuxième face 37B opposée, la première face 37A comprenant l'ouverture 39 dirigée vers la seconde branche 25' de la canule artérielle principale 22. L'ouverture permet de s'assurer que le sang peut circuler dans le tube, même si l'extrémité distale biseautée est obstruée. En effet, l'extrémité distale biseautée peut être obstruée par exemple par l'artère.

L'extrémité proximale 36 biseautée du tube 32, peut être disposée à proximité de la découpe 38. Elle peut être adaptée à la courbure du coude 27 de la canule artérielle principale 22 lorsque la canule de rétro-perfusion 30 est en position déployée. Ainsi, en position déployée, l'extrémité proximale 36 du tube est complémentaire du coude et ne dépasse pas de l'intérieur du coude, ce qui permet une circulation aisée du sang dans la canule artérielle principale.

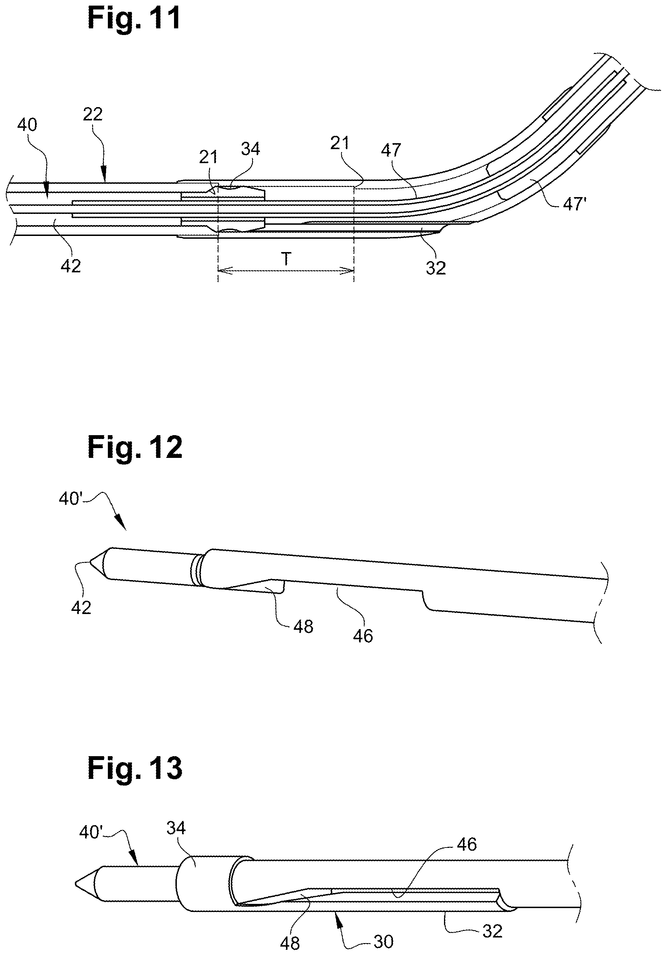

La canule artérielle principale 22 peut présenter une portion de translation T (figure 11) de la bague 34 de longueur identique à la longueur « l » du tube 32. La portion de translation T peut être disposée au niveau de la première extrémité 24 de la canule artérielle principale 22.

Le rapport entre le diamètre interne D' de la canule artérielle principale et le diamètre interne du tube 32 de la canule de rétro perfusion 30 peut être de 8.

La canule de rétro-perfusion 30 peut présenter une paroi présentant une épaisseur « e » de 0,1 mm.

Une lumière 26' (figure 3) telle qu'une lumière elliptique, peut être présente dans le coude 27 de la canule artérielle principale 22, à l'opposé de l'orifice de sortie 26 de sang, afin de permettre à la canule de rétro-perfusion 30 d'être déployée hors de la canule artérielle principale 22. La lumière 26' peut présenter une forme et des dimensions identiques à la forme et aux dimensions de la section transversale du tube 32. Ainsi, seul le tube 32 est apte à être déployé dans l'artère à travers la lumière 26'.

La figure 3 montre en outre que la première extrémité 24 de la canule artérielle principale 22 peut présenter une paroi interne 31 au contact de laquelle le tube 32 et la bague 34 de la canule de rétro-perfusion sont configurés pour glisser. Ainsi, le tube 32 peut être disposé au contact de la paroi interne 31.

En outre, le tube 32 de la canule de rétro perfusion 30 peut présenter un renflement 33 par exemple souple, configuré pour bloquer la canule de rétro-perfusion 30 en position déployée. Le renflement 33 peut être disposé sur une partie supérieure 32' du tube 32. Le renflement 33 peut être dirigée vers la deuxième extrémité 28 de la canule artérielle principale 22. Il peut coopérer avec la lumière 26' du coude 27. Plus précisément, il peut coopérer avec une partie externe et supérieure de la lumière 26' du coude 27 de la canule artérielle principale 22. Le renflement 33 permet d'empêcher un retrait spontané de la canule de rétro perfusion 30 dans la canule artérielle principale.

Le tube 32 peut être en matériau composite biocompatible. Il peut comporter une portion au niveau de la découpe 38 sous forme de feuillard en acier.

En outre, comme le montre la figure 4, la bague 34 peut comporter une nervure 52 configurée pour coopérer avec une rainure 50 (figure 6) disposée longitudinalement dans la paroi interne 31 de la canule artérielle principale 22. La rainure 50 peut présenter une longueur correspondant à la longueur de translation de la canule de rétro-perfusion 30. Ainsi, la rainure 50 permet de limiter et contenir la translation de la rétro canule. Elle permet d'empêcher la canule de rétro-perfusion 30 de s'échapper de la première extrémité 24 de la canule artérielle principale 22 lors du passage de la position rétractée à la position déployée, de telle sorte que l'extrémité proximale 36 du tube 32 s'arrête exactement dans l'épaisseur de la paroi du coude 27 de la canule artérielle principale 22 en position déployée. En outre, elle permet de limiter la translation de la canule de rétro-perfusion 30 lors du passage de la position déployée à la position rétractée, de telle sorte que l'extrémité distale 36' du tube 32 s'arrête exactement dans l'épaisseur de la paroi du coude 27 de la canule artérielle principale 22 en position rétractée.

Dans un mode de réalisation représenté à la figure 13, la bague 34 ne comporte pas de nervure 52 et la paroi interne 31 de la canule principale ne présente pas de rainure 50. Dans ce mode de réalisation, la canule artérielle principale présente, en dehors de la portion de translation T de la bague 34, un diamètre interne inférieur au diamètre D de la bague 34 (figure 11). Ainsi, la portion de translation T présentant un diamètre interne supérieur au diamètre interne du reste de la canule artérielle principale, le diamètre interne de la portion de translation T étant très légèrement supérieur au diamètre D de la bague. La translation de la bague est limitée lors du déploiement et/ou du retrait de la canule de rétro-perfusion par le diamètre interne inférieur de la canule artérielle principale hors de la portion de translation T de la bague. La différence de diamètre forme une butée 21 (figure 11) à chaque extrémité de la portion de translation T.

Comme le montre la figure 3, la canule artérielle principale peut présenter une protubérance 51 interne. Comme il sera vu par la suite, la protubérance 51 interne est configurée pour limiter la translation d'un mandrin d'introduction 40 destiné à être inséré dans la canule artérielle 20.

Les figures 7 à 11 illustrent un premier mode de réalisation d'un mandrin d'introduction 40 selon le présent exposé. Le mandrin d'introduction est configuré pour être inséré dans une canule artérielle 20 telle que décrite précédemment, avant son introduction dans une artère, afin de permettre à la fois l'insertion de la canule artérielle principale 22 dans l'artère et le déploiement de la canule de rétro-perfusion 30 hors de la canule artérielle principale 22. Il peut présenter une extrémité proximale 42 en forme de pointe, destinée à traverser l'orifice de sortie 26 de sang de la canule artérielle principale 22, lorsque le mandrin d'introduction 40 est inséré dans la canule artérielle, afin de faciliter l'introduction de la canule artérielle principale 22 dans l'artère. Il peut présenter en outre une extrémité distale 42' en forme de pince, configurée pour enserrer la deuxième extrémité 28 de la canule artérielle.

Comme le montre la figure 7, le mandrin d'introduction 40 peut présenter avantageusement une première portion intermédiaire 47, proximale à l'extrémité proximale 42, et une deuxième portion intermédiaire 47', proximale à l'extrémité distale 42' en forme de pince. La première portion intermédiaire 47 peut présenter une longueur légèrement supérieure à celle de la canule de rétro-perfusion. Elle peut présenter un diamètre inférieur au diamètre de la canule artérielle principale. Ainsi, la première portion intermédiaire 47 n'entre pas en conflit spatial avec la canule de rétro-perfusion lorsque celle-ci est en position rétractée dans la canule artérielle principale. La deuxième portion intermédiaire 47' en revanche peut présenter un diamètre sensiblement identique à celui de la canule artérielle principale afin de présenter un jeu minimal lors de son introduction dans la canule artérielle. En outre, ceci permet de conférer une certaine rigidité au mandrin d'introduction et de permettre une progression axiale et centrée lors de l'insertion du mandrin d'introduction dans la canule artérielle. Cependant, de façon à être non obstructive, la deuxième portion intermédiaire 47' peut présenter des rainures longitudinales (non représentées) permettant au sang de circuler.

L'extrémité proximale 42 du mandrin d'introduction 40 peut présenter un diamètre croissant, jusqu'à un diamètre maximal D" identique au diamètre interne D' de la canule artérielle principale 22, ce qui permet d'obstruer la canule artérielle principale 22. Ainsi, aucun flux de sang ne circule tant que le mandrin d'introduction est disposé dans la canule artérielle principale. Ce diamètre croissant permet au mandrin d'introduction 40 d'être introduit dans la première extrémité 24 de la canule artérielle principale 20 dans laquelle la canule de rétro-perfusion 30 est disposée en position rétractée. En effet, la canule de rétro-perfusion 30 en position rétractée réduit l'espace interne libre de la canule artérielle principale (comme cela peut être vu sur la figure 6).

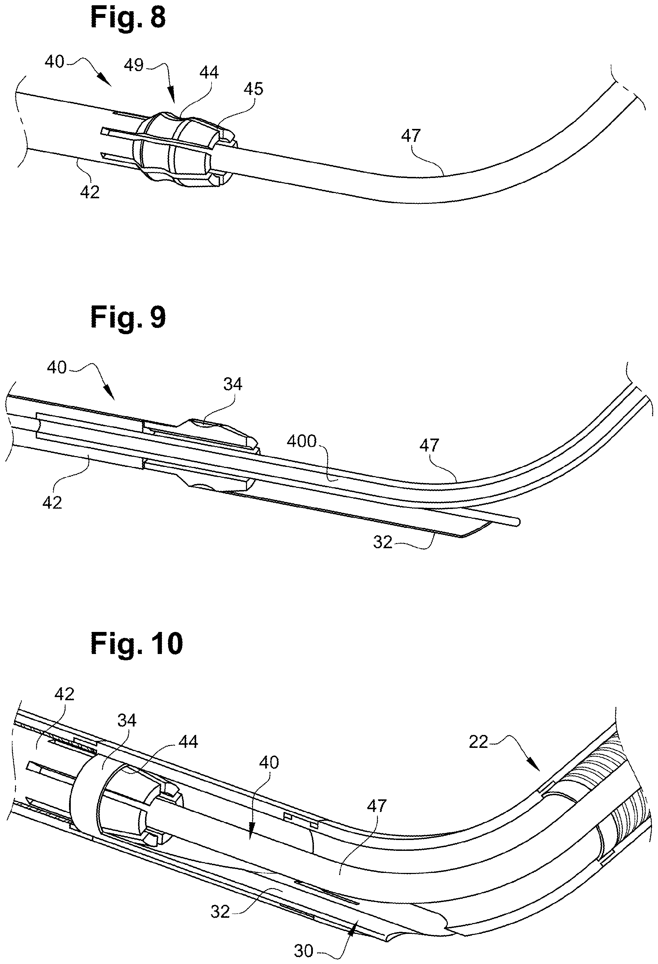

En outre, l'extrémité proximale 42 du mandrin d'introduction 40 peut être configurée pour coopérer avec la bague 34 de la canule de rétro-perfusion. A cet effet, l'extrémité proximale 42 peut comporter une gorge 44 dans laquelle la bague 34 est destinée à se loger (figure 10). La gorge 44 permet de maintenir la canule de rétro-perfusion 30 en position rétractée dans la canule artérielle principale 22 lors de l'insertion de la canule artérielle principale 22 dans une artère, et d'entrainer la canule de rétro-perfusion 30 dans sa position déployée lors du retrait du mandrin d'introduction 40 de la canule artérielle 20.

L'extrémité proximale 42 du mandrin d'introduction 40 peut être configurée pour entrer au butée contre la protubérance 51 (figure 3) interne de la canule artérielle principale, au niveau du diamètre maximal D". Ainsi, la translation du mandrin d'introduction 40 dans la canule artérielle principale est limitée de sorte que la bague 34 de la canule de rétro-perfusion se loge dans la gorge 44 du mandrin d'introduction.

La figure 8 montre que le mandrin d'introduction 40 peut présenter des incisures 45 au niveau et autour de la gorge 44. Ces incisures permettent de former un système rétentif 49 déformable. Ainsi, lorsque la canule de rétro-perfusion termine sa translation dans la canule artérielle principale lors de son déploiement, le diamètre du mandrin au niveau du système rétentif déformable, se rétracte légèrement lui permettant de glisser hors de la bague pour se libérer complétement de la canule artérielle.

Le mandrin d'introduction 40 peut être déformable, afin de pouvoir conférer toute forme à la canule artérielle principale 22. En effet, les parties souples de la canule artérielle principale 22 sont aptes à être déformées par le mandrin d'introduction 40, de sorte à lui donner par exemple une forme en Z comprenant une première branche libre correspondant à la première extrémité 24 destinée à être introduite dans une artère, une branche centrale et une seconde branche libre destinée à coopérer avec l'oxygénateur (non représenté).

Comme le montre la figure 9, le mandrin d'introduction 40 peut être perforé axialement en son centre en un cylindre 400 creux.

Les figures 12 et 13 illustrent un deuxième mode de réalisation d'un mandrin d'introduction 40' selon le présent exposé.

Le mandrin d'introduction 40' peut présenter un évidement 46 complémentaire du tube 32 de la canule de rétro-perfusion 30 (figure 10), permettant au mandrin d'introduction 40' d'être introduit dans la première extrémité 24 de la canule artérielle principale 20 dans laquelle la canule de rétro-perfusion 30 est disposée en position rétractée. Cet évidement 46 peut comporter un ergot 48 configuré pour coopérer avec le tube 32 de la canule de rétro-perfusion 30 afin de permettre son déploiement hors de la canule artérielle principale 22.

Le mandrin d'introduction 40 selon le premier mode de réalisation est symétrique par rapport à son axe longitudinal et est donc configuré pour s'introduire dans la canule artérielle 20 dans toute position quel que soit la rotation du mandrin sur son axe.

En revanche, le mandrin d'introduction 40' selon le deuxième mode de réalisation est configuré pour s'introduire dans la canule artérielle dans une position précise, de sorte que l'évidement 46 coopère avec le tube 32 de la canule de rétro-perfusion 30.

Ces mandrins 40, 40' permettent une translation bidirectionnelle de la canule de rétro-perfusion.

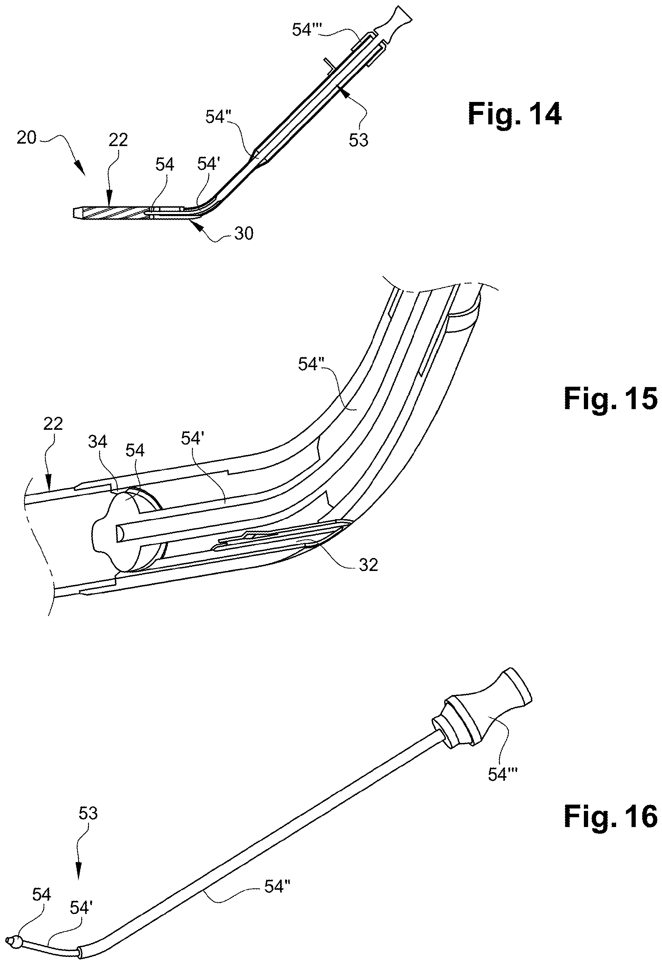

Les figures 14 à 16 illustrent un mandrin de retrait 53 configuré pour permettre de rétracter la canule de rétro-perfusion 30 dans la canule artérielle principale 22, afin de permettre de retirer de la canule artérielle principale 22 hors d'une artère.

Le mandrin de retrait 53 est configuré pour être inséré dans une canule artérielle 20 telle que décrite précédemment, lorsque la canule de rétro-perfusion 30 est en position déployée, afin de permettre de rétracter la canule de rétro-perfusion 30 dans la canule artérielle principale 22. Il peut présenter un renflement 54 configuré pour coopérer avec la bague 34 de la canule de rétro-perfusion 30 afin de l'entrainer en translation dans la canule artérielle principale 22, lorsque le mandrin de retrait 53 est inséré dans la canule artérielle principale 22. Le renflement 54 peut être disposé au niveau d'une extrémité dite proximale du mandrin de retrait 53. L'extrémité proximale du mandrin de retrait 53 est une extrémité destinée à être disposée à proximité de l'orifice de sortie 26 de sang de la canule artérielle principale 22, lorsque le mandrin de retrait 53 est inséré dans la canule artérielle 20. Le renflement 54 peut être destiné à s'encastrer dans la bague 34. Il peut obstruer la bague 34. Le renflement 54 peut être sphérique. Le mandrin de retrait 53 peut en outre présenter une première partie intermédiaire 54' de préférence cylindrique dont le diamètre permet le retrait de la canule de rétro-perfusion sans conflit spatial avec celle-ci lors de sa rétractation dans la canule artérielle principale. Cette première partie intermédiaire 54' peut présenter une longueur identique à celle de la canule de rétro-perfusion. Le mandrin de retrait peut présenter également une deuxième partie intermédiaire 54" de préférence cylindrique dont le diamètre est égal à celui de la canule artérielle principale afin de l'obstruer pour prévenir la remontée de sang dans la canule artérielle principale. Le mandrin de retrait peut présenter par ailleurs une extrémité distale 54‴ en forme de capuchon, configurée pour coopérer avec la deuxième extrémité 28 de la canule artérielle.

La deuxième partie intermédiaire 54" du mandrin de retrait 53 peut présenter un diamètre identique au diamètre interne D' de la canule artérielle principale 22, ce qui permet d'obstruer la canule artérielle principale 22. Ainsi, aucun flux de sang ne circule dans la canule artérielle lorsque le mandrin de retrait est disposé dans la canule artérielle principale.

Le renflement 54 est configuré pour coopérer avec la bague 34 qui peut présenter une paroi intérieure 34' convexe (figure 5) afin d'entrainer la canule de rétro-perfusion 30 en translation dans la canule artérielle principale 22, lors de son insertion dans la canule artérielle 20.

En outre, la paroi intérieure 34' convexe de la bague 34 permet de laminer le flux sanguin et ainsi d'éviter une hémolyse et des flux sanguins turbulents.

Comme le montre la figure 14 le mandrin de retrait 53 permet d'obstruer la canule artérielle principale 22 lorsqu'il est inséré dans la canule artérielle principale.

## Revendications

1. Canule artérielle (20) pour ECMO comprenant :
- une canule artérielle principale (22) comportant une paroi interne (31) et présentant une première extrémité (24) avec un orifice (26) de sortie de sang, la première extrémité étant destinée à être introduite dans une artère (100) afin d'injecter le sang de manière rétrograde dans l'artère, et
- une canule de rétro-perfusion (30) configurée pour être mobile en translation entre une position rétractée dans la première extrémité de la canule artérielle principale et une position déployée au moins en partie hors de la première extrémité de la canule artérielle principale, à l'opposé de l'orifice de sortie de sang de la canule artérielle principale, la canule de rétro-perfusion comprend un tube (32) de diamètre inférieur au diamètre de la canule artérielle principale et une bague (34) de diamètre sensiblement identique au diamètre de la canule artérielle principale, disposée à une extrémité proximale (35) de la canule de rétro-perfusion par rapport à l'orifice de sortie du sang de la canule artérielle principale.

2. Canule artérielle selon la revendication 1, présentant une forme en L avec une première branche (25) correspondant à la première extrémité (24) destinée à être introduite dans l'artère, et une seconde branche (25') destinée à coopérer avec un oxygénateur, la première branche et la seconde branche étant reliées par un coude (27).

3. Canule artérielle selon l'une quelconque des revendications précédentes, dans laquelle la canule de rétro-perfusion est configurée pour glisser en translation au contact de la paroi interne de la canule artérielle principale, jusqu'à ce que la bague entre en contact avec une butée (21) de la paroi interne.

4. Canule artérielle selon l'une quelconque des revendications précédentes, dans laquelle une découpe (38) est présente entre le tube et la bague.

5. Canule artérielle selon l'une quelconque des revendications précédentes, dans laquelle le tube présente un profil elliptique.

6. Canule artérielle selon l'une quelconque des revendications précédentes, dans laquelle la bague présente une paroi intérieure (34') convexe.

7. Canule artérielle selon l'une quelconque des revendications précédentes, dans laquelle la canule artérielle principale présente une rainure longitudinale (50) au niveau de sa paroi interne coopérant avec une nervure (52) disposée sur la bague de la canule de rétro-perfusion.

8. Canule artérielle selon l'une quelconque des revendications précédentes, dans laquelle la canule artérielle principale présente une portion de translation (T) de la bague (34), la portion de translation présentant un diamètre interne supérieur au diamètre interne du reste de la canule artérielle principale.

9. Canule artérielle selon l'une quelconque des revendications 2 à 8, en combinaison avec la revendication 2, dans laquelle le tube (32) présente une extrémité distale (36') biseautée, à l'opposé de la bague (34), de sorte à être adaptée à la courbure du coude (27) de la canule artérielle principale (22) lorsque la canule de rétro-perfusion (30) est en position rétractée, le tube (32) comportant une ouverture (29) dirigée vers la seconde branche (25') de la canule artérielle principale (22).

10. Mandrin d'introduction (40, 40') configuré pour être inséré dans la canule artérielle selon l'une quelconque des revendications précédentes, afin de permettre à la fois l'insertion de la canule artérielle principale dans une artère et le déploiement de la canule de rétro-perfusion hors de la canule artérielle principale, le mandrin d'introduction étant configuré pour obstruer la canule artérielle principale.

11. Mandrin d'introduction (40, 40') selon la revendication précédente, comportant une extrémité proximale (42) configurée pour être disposée dans la première extrémité de la canule artérielle principale, l'extrémité proximale (42) présentant un diamètre croissant jusqu'à un diamètre sensiblement identique au diamètre de la canule artérielle principale, l'extrémité proximale (42) étant suivie d'une première portion intermédiaire (47) de longueur au moins supérieure à celle de la canule de rétro-perfusion et de diamètre inférieur au diamètre de la canule artérielle principale, la première portion intermédiaire (47) étant suivie d'une deuxième portion intermédiaire (47') de diamètre sensiblement identique à celui de la canule artérielle principale.

12. Mandrin d'introduction (40, 40') selon l'une quelconque des revendications 10 à 11, présentant une forme complémentaire de la canule artérielle principale avec la canule de rétro-perfusion en position rétractée.

13. Mandrin de retrait (53) configuré pour être inséré dans la canule artérielle selon l'une quelconque des revendications 1 à 9, afin de permettre de rétracter la canule de rétro-perfusion dans la canule artérielle principale, et de permettre de retirer la canule artérielle principale hors de l'artère, le mandrin de retrait étant configuré pour obstruer la canule artérielle principale.

14. Mandrin de retrait (53) selon la revendication précédente, comprenant un renflement (54) configuré pour coopérer avec la bague (34) de la canule de rétro-perfusion (30) afin de l'entrainer en translation dans la canule artérielle principale (22), lorsque le mandrin de retrait (53) est inséré dans la canule artérielle principale.

15. Kit comprenant une canule artérielle selon l'une quelconque des revendications 1 à 9, un mandrin d'introduction selon l'une quelconque des revendications 10 à 12 et un mandrin de retrait selon l'une quelconque des revendications 13 à 14.

## Patentansprüche

1. Arterienkanüle (20) für ECMO, umfassend:
- eine Hauptarterienkanüle (22), die eine Innenwand (31) umfasst und ein erstes Ende (24) mit einer Blutauslassöffnung (26) aufweist, wobei das erste Ende dazu bestimmt ist, in eine Arterie (100) eingeführt zu werden, um Blut retrograd in die Arterie zu injizieren, und
- eine Retroperfusionskanüle (30), die dazu konfiguriert ist, translatorisch zwischen einer zurückgezogenen Position im ersten Ende der Hauptarterienkanüle und einer ausgefahrenen Position mindestens teilweise aus dem ersten Ende der Hauptarterienkanüle, gegenüberliegend der Blutauslassöffnung der Hauptarterienkanüle, beweglich zu sein, wobei die Retroperfusionskanüle eine Hülse (32) mit einem Durchmesser, der kleiner ist als der Durchmesser der Hauptarterienkanüle, und einen Ring (34) mit einem Durchmesser umfasst, der im Wesentlichen identisch mit dem Durchmesser der Hauptarterienkanüle ist und in Bezug auf die Blutauslassöffnung der Hauptarterienkanüle an einem proximalen Ende (35) der Retroperfusionskanüle angeordnet ist.

2. Arterienkanüle nach Anspruch 1, die eine L-Form mit einem ersten Schenkel (25) aufweist, der dem ersten Ende (24) entspricht, das dazu bestimmt ist, in die Arterie eingeführt zu werden, und einen zweiten Schenkel (25'), der dazu bestimmt ist, mit einem Oxygenator zusammenzuwirken, wobei der erste Schenkel und der zweite Schenkel durch einen Winkel (27) verbunden sind.

3. Arterienkanüle nach einem der vorhergehenden Ansprüche, wobei die Retroperfusionskanüle dazu konfiguriert ist, dass in Kontakt mit der Innenwand der Hauptarterienkanüle translatorisch zu gleiten, bis der Ring mit einem Anschlag (21) der Innenwand in Kontakt kommt.

4. Arterienkanüle nach einem der vorhergehenden Ansprüche, wobei zwischen der Hülse und dem Ring eine Aussparung (38) vorhanden ist.

5. Arterienkanüle nach einem der vorhergehenden Ansprüche, wobei die ein elliptisches Profil aufweist.

6. Arterienkanüle nach einem der vorhergehenden Ansprüche, wobei der Ring eine konvexe Innenwand (34') aufweist.

7. Arterienkanüle nach einem der vorhergehenden Ansprüche, wobei die Hauptarterienkanüle eine Längsnut (50) auf Höhe ihrer Innenwand aufweist, die mit einer Wulst (52) zusammenwirkt, die auf dem Ring der Retroperfusionskanüle angeordnet ist.

8. Arterienkanüle nach einem der vorhergehenden Ansprüche, wobei die Hauptarterienkanüle einen Translationsabschnitt (T) des Rings (34) aufweist, wobei der Translationsabschnitt einen Innendurchmesser aufweist, der größer ist als ein Innendurchmesser des Rests der Hauptarterienkanüle.

9. Arterienkanüle nach einem der Ansprüche 2 bis 8, in Kombination mit Anspruch 2, wobei der Schlauch (32) gegenüber dem Ring (34) ein abgeschrägtes distales Ende (36') aufweist, um an eine Krümmung des Winkelstücks (27) der Hauptarterienkanüle (22) angepasst zu werden, wenn sich die Retroperfusionskanüle in der zurückgezogenen Position befindet, wobei der Schlauch (32) eine Öffnung (29) umfasst, die in Richtung des zweiten Schenkels (25') der Hauptarterienkanüle (22) gerichtet ist.

10. Einführungsmandrin (40, 40'), der dazu konfiguriert ist, in die Arterienkanüle nach einem der vorhergehenden Ansprüche eingeführt zu werden, um sowohl das Einführen der Hauptarterienkanüle in eine Arterie als auch das Ausfahren der Retroperfusionskanüle aus der Hauptarterienkanüle zu gestatten, wobei der Einführungsmandrin dazu konfiguriert ist, die Hauptarterienkanüle zu blockieren.

11. Einführungsmandrin (40, 40') nach dem vorhergehenden Anspruch, umfassend ein proximales Ende (42), das dazu konfiguriert ist, im ersten Ende der Hauptarterienkanüle angeordnet zu werden, wobei das proximale Ende (42) einen Durchmesser aufweist, der bis zu einem Durchmesser zunimmt, der im Wesentlichen identisch mit dem Durchmesser der Hauptarterienkanüle ist, wobei auf das proximale Ende (42) ein erster Zwischenabschnitt (47) mit einer Länge, die mindestens größer ist als die der Retroperfusionskanüle, und einem Durchmesser, der kleiner ist als der Durchmesser der Hauptarterienkanüle, folgt, wobei auf den ersten Zwischenabschnitt (47) ein zweiter Zwischenabschnitt (47') mit einem Durchmesser folgt, der im Wesentlichen identisch mit dem der Hauptarterienkanüle ist.

12. Einführungsmandrin (40, 40') nach einem der Ansprüche 10 bis 11, aufweisend eine zur Hauptarterienkanüle komplementäre Form, wobei sich die Retroperfusionskanüle in der zurückgezogenen Position befindet.

13. Rückzugsmandrin (53), der dazu konfiguriert ist, in eine Arterienkanüle nach einem der Ansprüche 1 bis 9 eingeführt zu werden, um das Zurückziehen der Retroperfusionskanüle in die Hauptarterienkanüle zu gestatten und das Entfernen der Hauptarterienkanüle aus der Arterie zu gestatten, wobei der Rückzugsmandrin dazu konfiguriert ist, die Hauptarterienkanüle zu blockieren.

14. Rückzugsmandrin (53) nach dem vorhergehenden Anspruch, umfassend eine Ausbuchtung (54), die dazu konfiguriert ist, mit dem Ring (34) der Retroperfusionskanüle (30) zusammenzuwirken, um diese in der Hauptarterienkanüle (22) translatorisch anzutreiben, wenn der Rückzugsmandrin (53) in die Hauptarterienkanüle eingeführt wird.

15. Kit, umfassend eine Arterienkanüle nach einem der Ansprüche 1 bis 9, einen Einführungsmandrin nach einem der Ansprüche 10 bis 12 und einen Rückzugsmandrin nach einem der Ansprüche 13 bis 14.

## Claims

1. An arterial cannula (20) for ECMO comprising:
- a main arterial cannula (22) including an inner wall (31) and having a first end (24) with a blood outlet orifice (26), the first end being intended to be inserted into an artery (100) in order to inject blood in a retrograde manner into the artery, and
- a retroperfusion cannula (30) configured to be movable in translation between a retracted position in the first end of the main arterial cannula and a position at least partially deployed out of the first end of the main arterial cannula, opposite to the blood outlet orifice of the main arterial cannula, the retroperfusion cannula comprises a tube (32) with a diameter smaller than the diameter of the main arterial cannula and a ring (34) with a diameter substantially identical to the diameter of the main arterial cannula, disposed at a proximal end (35) of the retroperfusion cannula with respect to the blood outlet orifice of the main arterial cannula.

2. The arterial cannula according to claim 1, having a L-shape with a first branch (25) corresponding to the first end (24) intended to be introduced into the artery, and a second branch (25') intended to cooperate with an oxygenator, the first branch and the second branch being connected by an elbow (27).

3. The arterial cannula according to any one of the preceding claims, wherein the retroperfusion cannula is configured to slip in translation in contact with the inner wall of the main arterial cannula, until the ring comes into contact with a stop (21) of the inner wall.

4. The arterial cannula according to any one of the preceding claims, wherein a cutout (38) is present between the tube and the ring.

5. The arterial cannula according to any one of the preceding claims, wherein the tube has an elliptical profile.

6. The arterial cannula according to any one of the preceding claims, wherein the ring has a convex internal wall (34').

7. The arterial cannula according to any one of the preceding claims, wherein the main arterial cannula has a longitudinal groove (50) at its inner wall cooperating with a rib (52) disposed on the ring of the retroperfusion cannula.

8. The arterial cannula according to any one of the preceding claims, wherein the main arterial cannula has a translation portion (T) of the ring (34), the translation portion having an inner diameter larger than the inner diameter of the rest of the main arterial cannula.

9. The arterial cannula according to any one of claims 2 to 8, in combination with claim 2, wherein the tube (32) has a beveled distal end (36'), opposite to the ring (34), so as to be adapted to the curvature of the elbow (27) of the main arterial cannula (22) when the retroperfusion cannula (30) is in the retracted position, the tube (32) including an opening (29) directed towards the second branch (25') of the main arterial cannula (22).

10. An introducer stylet (40, 40') configured to be inserted into the arterial cannula according to any one of the preceding claims, in order to enable both the insertion of the main arterial cannula into an artery and the deployment of the retroperfusion cannula out of the main arterial cannula, the introducer stylet being configured to obstruct the main arterial cannula.

11. The introducer stylet (40, 40') according to the preceding claim, including a proximal end (42) configured to be disposed in the first end of the main arterial cannula, the proximal end (42) having a diameter increasing up to a diameter substantially identical to the diameter of the main arterial cannula, the proximal end (42) being followed by a first intermediate portion (47) with a length at least larger than that of the retroperfusion cannula and a diameter smaller than the diameter of the main arterial cannula, the first intermediate portion (47) being followed by a second intermediate portion (47') with a diameter substantially identical to that of the main arterial cannula.

12. The introducer stylet (40, 40') according to any one of claims 10 to 11, having a shape complementary to the main arterial cannula with the retroperfusion cannula in the retracted position.

13. A removal stylet (53) configured to be inserted into the arterial cannula according to any one of claims 1 to 9, in order to allow retracting the retroperfusion cannula in the main arterial cannula, and to allow removing the main arterial cannula out of the artery, the removal stylet being configured to obstruct the main arterial cannula.

14. The removal stylet (53) according to the preceding claim, comprising a bulge (54) configured to cooperate with the ring (34) of the retroperfusion cannula (30) in order to drive it in translation in the main arterial cannula (22), when the removal stylet (53) is inserted into the main arterial cannula.

15. A kit comprising an arterial cannula according to any one of claims 1 to 9, an introducer stylet according to any one of claims 10 to 12 and a removal stylet according to any one of claims 13 to 14.
